(19) Europäisches Patentamt European Patent Office Office européen des brevets

(11) **EP 1 756 168 B1**

(12) **EUROPEAN PATENT SPECIFICATION**

(45) Date of publication and mention
of the grant of the patent:
**29.07.2009 Bulletin 2009/31**

(21) Application number: **05743005.0**

(22) Date of filing: **30.03.2005**

(51) Int Cl.:
***C08F 2/00*** (2006.01)

(86) International application number:
**PCT/EP2005/051429**

(87) International publication number:
**WO 2005/097834 (20.10.2005 Gazette 2005/42)**

(54) **LIQUID DISPERSION POLYMER COMPOSITIONS, THEIR PREPARATION AND THEIR USE**

FLÜSSIGE DISPERSIONSPOLYMERZUSAMMENSETZUNGEN, IHRE HERSTELLUNG UND IHRE VERWENDUNG

COMPOSITIONS POLYMERES DE DISPERSION LIQUIDE, LEUR PREPARATION ET LEUR UTILISATION

(84) Designated Contracting States:
**AT BE BG CH CY CZ DE DK EE ES FI FR GB GR HU IE IS IT LI LT LU MC NL PL PT RO SE SI SK TR**

(30) Priority: **06.04.2004 EP 04101419**

(43) Date of publication of application:
**28.02.2007 Bulletin 2007/09**

(73) Proprietor: **Ciba Holding Inc.**
**4057 Basel (CH)**

(72) Inventors:
• **RIDLEY, Eleanor Bernice**
**Leeds,**
**West Yorkshire LS12 5SZ (GB)**
• **GREEN, Michael**
**Huddersfield,**
**West Yorkshire HD7 5XA (GB)**
• **NORMINGTON, David**
**Leeds West,**
**West Yorkshire LS28 8PB (GB)**

(56) References cited:
**US-A- 4 677 152**

**Description**

The present invention relates to liquid dispersion polymer compositions that comprise a dispersed polymer phase, a continuous carrier phase and a surfactant. U.S. 4,677,152 teaches the preparation of an aqueous dispersion starting from a polymer-in-water emulsion and subsequent emulsification into a non-aqueous liquid.

Thickeners are used extensively in personal care formulations such as cosmetic and pharmaceutical formulations, to affect the aesthetics, product application and suspension and delivery of the active raw materials. Polymeric thickeners have been used for this purpose for many years. The types of polymeric thickeners that have been used range from natural gums such as guar, through modified natural materials such as hydroxyethyl cellulose, to synthetic thickeners such as the Carbomers® based on polyacrylic acids.

The Salcare® and Tinovis® ranges of liquid dispersion polymers, available through Ciba Specialty Chemicals, are ranges of microparticulate acrylic-based polymeric thickeners in a hydrophobic carrier medium. Salcare® SC91 is an anionic thickening agent based on a sodium acrylate polymer and mineral oil carrier with PPG-1 trideceth-6 as the activator surfactant. Salcare® SC92 is a cationic copolymer thickener and conditioner comprising polyquaternium 32 and mineral oil. Salcare® SC95 and Salcare® SC96 are cationic homopolymer thickeners and conditioners. Salcare® SC95 comprises polyquaternium 37 in mineral oil with PPG-1 trideceth-6. Salcare® SC96 comprises polyquaternium 37 in propylene glycol dicaprylate dicaprate with PPG-1 trideceth-6. Salcare® AST is an anionic thickening agent based on a sodium acrylate polymer in soya bean oil with PPG-1 trideceth-6.

The tiny, spherical microparticles of the above hydrophilic acrylic polymers, whether anionic or cationic in charge, have a typical particle size in the range of 0.1 - 2 microns, with an average particle size in the range of 0.5 - -1.0 microns. The polymer microparticles are preferably manufactured by methods in which water-soluble vinyl addition monomers are polymerized utilizing a water-in-oil polymerization route.

On stirring of any of the above liquid dispersion polymers into an aqueous system, the activator surfactant converts the hydrophobic carrier into an oil-in-water emulsion. By the term "activator surfactant" is meant a surfactant that activates the conversion of the hydrophobic carrier into an oil-in-water emulsion. At the same time the hydrophilic polymer expands on exposure to water but does not dissolve, resulting in a smooth and rapid viscosity increase. Typically the polymer particles swell to give a microparticulate thickening system comprising polymer particles having a typical particle size in the range of 2.5 - 5 microns in diameter. Since the water molecules move into the small polymer particles by osmosis, the osmotic effect experienced by the polymer particle is a balance between water and any electrolyte present in the system. Hence high electrolyte levels reduce the swelling of the polymer particles.

The microparticulate thickening systems have a pseudoplastic rheological profile which gives good stability and suspension characteristics at low shear rates (such as those experienced by the product on standing), and low apparent viscosities at high shear rates, which corresponds to excellent rub-in characteristics.

Surprisingly, it has been found out that the use of a polymer with a neutralization level of from about 25 to about 50 % in the process leads to improved thickened systems.

The level of neutralization is defined as

$$\frac{\Sigma \ \text{neutralized sites of the polymer}}{\Sigma \ \text{neutralizable sites of the polymer}} \ \times \ 100\%$$

The level of neutralization goes from 0%, wherein no neutralizable site of the polymer is neutralized up to 100%, wherein all neutralizable sites of the polymer are neutralized.

The level of neutralization is measured by conventional acid-base titration to determine the % converted neutralisable sites.

In one aspect the present invention provides a liquid dispersion polymer composition comprising a hydrophilic, water soluble or swellable polymer with a neutralization level of from about 25% to about 100 %, preferably from about 30% to about 50 %, more preferred from about 30% to about 45%, especially from about 30% to about 40%, a non-aqueous carrier phase and an oil-in-water surfactant.

Preferably, the hydrophilic, water soluble or swellable polymer obtained by the process is used in the form of microparticles having an average particle size in the range of about 0.1 to about 2 microns.

Typically the liquid dispersion polymer composition obtained by the process comprises

a) from 35% to 65% by weight of the polymer with a neutralization level of from about 25% to about 50 %, preferably from about 30% to about 50 %, more preferred from about 30% to about 45%, especially from about 30% to about 40%,

b) from 20% to 50% by weight of a non-aqueous carrier and
c) from 5% to 25% by weight of a surfactant or a surfactant mixture,

each based on the total weight of the composition.

Advantageously the hydrophilic polymer a) is water swellable, i.e. it is sufficiently cross-linked to swell but not dissolve in water. Preferably it is acrylic-based. Also it is preferably anionic.

The non-aqueous carrier fluids mentioned in b) are well known to the cosmetic industry and have been used for many years in hair and skin formulations. They include silicone polymers, mineral oil, hydrogenated polydecene, isohexadecane, esters such as trimethyloylpropane tricapryleltricaprylate, $C_{12}$-$C_{15}$alkyl benzoate, ethylhexyl stearate, caprylic capric triglycerides, squalane, ethylhexyl cocoate, decyl oleate, decyl cocoate, ethyl oleate, isopropyl myristate, ethylhexyl perlagonate, pentaerythrityl tebacaprylateltetracaprate, PPG-3 benzyl ether myristate, propylene glycol dicaprylate / dicaprate, ethylhexyl isostearate, ethylhexyl palmitate and natural oils such as glycine soja, helianthus annuus, simmondsia chinensis, carthamus tinctorius, oenothera biennis and rapae oleum raffinatum as well as mixtures and derivatives of all these compounds.

Preferably the surfactant mixture c) comprises both surfactants useful in the manufacture of the microparticles of swellable polymer a), and at least one surfactant which serves as the activator for the subsequent oil-in-water microparticulate thickening emulsions. This activator surfactant for the oil-in-water thickening emulsions comprises from 1.0% to 10.0% by weight of the composition, preferably from 2.0% to 8.0% by weight of the composition. Preferably the activator surfactant is a nonionic oil-in-water emulsifier having a HLB generally above 7. Suitable emulsifiers of this type are well known to those skilled in the art. Ethoxylated alcohols are preferred.

Additionally the composition may contain minor amounts of other components which do not affect its essential characteristics. Typically these other components may include up to about 3% by weight each of water and volatile organic solvents as well as small amounts of other components which are left over from the preparation of the water soluble or swellable polymers.

Advantageously the composition (A) obtained by the process of the invention comprises

a) from 40% to 60% by weight of the polymer with a neutralization level of from about 25% to about 50 %, preferably from about 30% to about 50 %, more preferred from about 30% to about 45%, especially from about 30% to about 40%, wherein the polymer is anionic and is water swellable,
b) from 25% to 45% by weight of a non-aqueous carrier fluid, and
c) from 8% to 20% by weight of a surfactant or a surfactant mixture,

each based on the total weight of the composition.

A particularly preferred composition (B) obtained by the process of the invention comprises

a) from 45% to 58% by weight of the polymer with a neutralization level of from about 25% to about 50 %, preferably from about 30% to about 50 %, more preferred from about 30% to about 45%, especially from about 30% to about 40%, wherein the polymer is anionic and is water swellable,
b) from 30% to 40% by weight of a non-aqueous carrier fluid and
c) from 10% to 18% by weight of a mixture of surfactants,

each based on the total weight of the composition.

A very particularly preferred composition (C) obtained by the process of the invention comprises

a) from 45% to 58% by weight of the water swellable polymer with a neutralization level of from about 25% to about 50 %, preferably from about 30% to about 50 %, more preferred from about 30% to about 45%, especially from about 30% to about 40%, wherein the polymer is anionic,
b) from 32% to 38% by weight of a non-aqueous carrier fluid
c) from 12% to 18% by weight of a mixture of surfactants,

each based on the total weight of the composition.

Thickened aqueous or water-containing compositions (D), in particular personal care formulations, comprise

a) 0.1 % to 8% by weight, preferably 1% to 6% by weight of a liquid dispersion polymer composition (A), (B) or (C) as described above,
b) 0.1 % to 70%, preferably 2% to 35% by weight of additional ingredients, for example personal care ingredients such as cosmetic or pharmaceutical excipients and/or active ingredients and
c) 45% to 99% of water or a mixture of water and a inrater-miscible organic solvent such as a lower alcohol,

each based on the total weight of the composition.

Such lower alcohols include ethanol, isopropyl alcohol, propylene glycol, di-isopropyl alcohol and other known lower alcohols.

The compositions are useful for further processing to non-aqueous liquid or solid compositions, such as colour cosmetics or soaps, where the polymer of the present invention provides some additional benefit, such as improved wear properties.

These compositions may be in the form of lotions, creams, salves, gels, milks, sprays, foams or ointments.

The additional components can be any ingredient that may form part of a thickened aqueous emulsion of the oil-in-water type. Non-limiting examples of cosmetic ingredients include: antimicrobials (such as triclosan or famesol); skin conditioning agents and emollients such as lanolin and derivatives thereof; esters such as iso-propyl propanoate, decyl oleate, isopropyl isostearate, triactanoin, triisostearin, myristyl propionate; fatty alochols; squalene; silicones such as cyclomethicone, dimethicone, dimethicone copolyol; acetamide monoethanolamine; dimethyl polysiloxane; moisturizers such as aloe vera, barrier creams, emollients, alpha and beta hydroxy acids such as lactic acid and glycolic acid; anti-inflammatory actives like allantoin and bisabolol; UV sun screening agents such as para aminobenzoic acid, octyl salicylate, and octyl methoxycinnamate, "sunless" tanning agents, whitening agents, insect repellents, essential oils such as patchouli oil, peppermint oil, rosemary oil, citronella oil, tea tree oil, orange or lemon oils, cedarwood oil and sandalwood oil, vitamins, colours and pigments; hair conditioners such as amodimethicone, cyclomethicone, panthenol, lauramide diethanolamine, lauramine oxide and silk protein; perfume components; hair dyes and bleaches and preservatives such as methyl-, ethyl-, propyl- paraben and imidazolidinyl urea. Pharmaceutically active ingredients may vary widely and include all therapeutic agents intended for topical application to the skin or hair, in particular substances to treat itching, tingling, scaling, inflammation or infection of the skin, bums, and scalp hair loss of humans or other mammals.

A therapeutic lotion, cream, salve, gel or ointment is prepared by mixing 0-1% to 8% by weight, preferably 1% to 6% by weight of a liquid dispersion polymer as described above into an aqueous or aqueous/organic composition which contains from 0.1% to 70% by weight of at least one therapeutic agent and/or excipient.

These compositions are applicable in a method for the topical treatment of the skin or hair, which comprises applying a composition as defined above to the skin, face, hair or scalp of a human being or other mammal in need of such treatment. The type of treatment will depend on the active ingredient(s) dissolved or suspended in the composition. For example the composition may comprise facial creams such as barrier cream, a moisturizer cream, lotion or milk, a cleanser or toner, a hand and body milk or lotion, a body spray, creams, lotions or milks containing sun-screens against UV-A and UV-B radiation, a "sunless" tanning cream, lotion or spray, a skin bleaching cream, lotion or spray, a depilatory cream, a hair conditioning cream, lotion or shampoo, a hair dyeing cream or lotion, a pre- or aftershave cream, lotion, gel or balm, a disinfectant cream, lotion, ointment or gel, a soothing cream, lotion or spray as an after sun application for sunburn, etc..

Other aspects of the present invention will become apparent from the following discussion and the examples. The examples merely illustrate certain aspects of the invention and are not intended to be limiting thereof.

The hydrophilic, water swellable acrylic-based liquid dispersion polymer compositions employed in the present invention are overall anionic in character. Said polymers with a neutralization level of from about 25% to about 50 % may be homopolymers or copolymers. They are formed from one or more monoethylenically unsaturated monomers that are either water soluble anionic monomers or from a predominantly anionic blend of monomers that may consist of a mixture of anionic monomers and a minor amount of nonionic monomers. The polymers with a neutralization level of from about 25% to about 50 % may conveniently be obtained in the form of microparticles having an average particle size in the range of 0.1 - 2 microns by reverse phase emulsion polymerization of suitable monomers in a hydrophobic liquid, i.e. a liquid which has sufficiently low miscibility with water that it can be used as the non-aqueous phase in a reverse phase polymerization. The liquid must have substantially no solvating effect for the polymer, or for the monomers from which it is formed, throughout the range of temperatures at which the polymer is likely to be synthesized (for instance from 15 to 100°C), since a solvating medium would be unsatisfactory for reverse phase emulsion polymerization. Likewise, the monomer or monomer blend must be water soluble to enable reverse phase polymerization to be carried out.

Suitable anionic monomers include acrylic acid, methacrylic acid and their alkali metal and ammonium salts, 2-acrylamido-2-methyl-propanesulfonic acid and its salts, sodium styrene sulfonate and the like. Acrylic acid is the most preferred anionic monomer. Preferably the carboxylic acid groups are between 20 and 80%, advantageously 30-45% in the form of an alkali metal salt or ammonium salt, especially the sodium salt

Suitable nonionic monomers include acrylamide, methacrylamide, N,N,-dialkylacrylamides, N-vinyl pyrrolidone and water soluble hydroxy-substituted acrylic or methacrylic esters.

If an anionic blend is used, the amount of anionic monomer is preferably more than 60% by weight of the blend, and usually it is at least 80% by weight of the blend. The preferred anionic polymers are formed wholly from anionic monomers.

The liquid dispersion polymer compositions are advantageously crosslinked by incorporating a small amount of a suitable crosslinking agent such as a polyfunctional vinyl addition monomer into the polymerization mixture. Preferably a water-soluble crosslinking agent is used.

Any of the conventional ethylenically unsaturated cross linking agents or poly ethylenically unsaturated cross linking agents which are soluble in the monomer or monomer blend can be used, including materials which are di-, tri- or tetraethylenically unsaturated. Preferred are diethylenically unsaturated compounds such as methylene bis acrylamide, di (meth)acrylamide, triallyl ammonium salts, vinyloxyethylacrylate or-methacryiate, divinyl benzene; tetra allyl ammonium chloride; allyl acrylates and methacrylates; diacrylates and dimethacrylates of glycols and polyglycols; butadiene; 1,7-octadiene; allyl-acrylamides and allyl-methacrylamides; bisacrylamidoacetic acid; N,N'-methylene-bisacrylamide and polyol polyallylethers, such as polyallylsaccharose and pentaerythrol triallylether.

More preferred cross-linking agents are tetra allyl ammonium chloride; allyl-acrylamides and allyl-methacrylamides; bisacrylamidoacetic acid and N,N'-methylene-bisacrylamide.

The most preferred cross-linking agents are tetra allyl ammonium chloride and N,N'-methylene-bisacrylamide.

It is also suitable to use mixtures of cross-linking agents.

The amount of cross linking agent is generally in the range from 100 to 10,000 parts by weight of cross linking agent per million parts (by dry weight) of monomer. Most preferably it is around 500 to 2000 ppm, especially 500 to 900 ppm for either cationic or anionic monomers. Optimum amounts can be determined by routine experimentation.

The hydrophilic polymers are prepared by reverse phase emulsion polymerization of hydrophilic monomers, preferably one or more acrylate and/or methacrylate monomers, in a hydrophobic liquid phase. Reverse phase emulsion polymerization is a well known technique which is described for example in U.S. Patent 4,628,078, the disclosure of which is incorporated by reference in its entirety.

The continuous phase for the preparation of the instant liquid dispersion polymer compositions is provided at least in part by a non-aqueous carrierfluid. Since the liquid dispersion polymer compositions are primarily intended for cosmetic or pharmaceutical purposes, non-aqueous carrier fluids which are cosmetically and/or pharmaceutically acceptable and which are sufficiently hydrophobic to be useful as the continuous phase in a reverse phase polymerization are preferably used as the continuous phase. Many such materials are known and are commercially available. Such non-aqueous carrier fluids include mineral oil, hydrogenated polydecene, natural oils including soya bean oil, Squalane, emollient esters including propylene glycol dicaprate caprylate. The preferred non-aqueous carrier fluid is mineral oil.

The amount of the hydrophobic liquid phase used in the polymerization is dictated primarily by the need to provide a satisfactory reverse phase emulsion medium. This would generally be at least about 0.5 parts by weight of the non-aqueous carrier fluid per part by weight of the hydrophilic polymer (dry weight). In order to obtain liquid dispersion polymer compositions having higher amounts of the microparticles in the non-aqueous carrier fluid, for example from 1.2 to about 1.7 parts by weight of the hydrophilic polymer (dry weight) in the non-aqueous carrier fluid, as well as to facilitate processing, it is expedient to employ a volatile inert hydrophobic solvent. Suitable inert hydrophobic solvents include hydrocarbons and halogenated hydrocarbons. One particularly preferred hydrocarbon mixture is Isopar G® (Trademark of Exxon Mobil Corporation). Conveniently 1 to 2 parts, preferably 1.3 to 1.9 parts of the volatile inert hydrophobic solvent per part of the hydrophilic polymer on a dry weight basis is employed.

The polymer is prepared by conventional reverse phase emulsion procedures, namely by adding 1 part by weight (dry weight) of at least one aqueous ethylenically unsaturated monomer, optionally including a sequesterant and a crosslinking ethylenically unsaturated monomer, into about 1 to 3 parts by weight of a hydrophobic liquid comprising at least in part a non-aqueous carrier fluid and containing about 0.1 to 0.2 parts of at least one conventional water-in-oil emulsifier having a HLB value below 9.0 and optionally 0.5 to 10.0 parts of a polymeric stabilizer surfactant, with intensive agitation so as to form a substantially stable emulsion of the required fine particle size. Suitable water-in-oil emulsifiers are well known to those skilled in the art. Sorbitan esters are preferred. Diethylenetriamine pentaacetic acid, sodium salt is a suitable sequesterant. The ethylenically unsaturated monomer may be diethylenically or polyethylenically unsaturated.

The reaction mixture is purged with nitrogen and polymerization is initiated by addition of a conventional source of free radicals. Suitable polymerization initiators are well known to those skilled in the art. Typical free radical-forming catalysts include peroxygen compounds such as sodium, potassium and ammonium persulfates, caprylyl peroxide, benzoyl peroxide, hydrogen peroxide, pelargonyl peroxide, cumene hydroperoxide, tertiary butyl diperphthalate, tertiary butyl perbenzoate, sodium peracetate, di(2-ethylhexyl)peroxydicarbonate, and the like, as well as azo catalysts such as azodiisobutyronitrile. Other useful catalysts are the heavy metal-activated catalyst systems. A preferred type of polymerization initiator is a redox initiation pair. After initiation appropriate temperature and agitation conditions are maintained until the conversion of the monomer to polymer is substantially complete. Appropriate conditions are well known to those of ordinary skill in the art.

The water and any volatile solvent are then removed from the reverse phase emulsion, for example by distillation under reduced pressure, so as to produce a substantially anhydrous stable dispersion of polymer particles less than 2 microns in size dispersed in the non-aqueous continuous phase.

About 1.0% to 10.0% by weight, based on the weight of the composition, preferably from 2% to 8% by weight of a nonionic oil-in-water emulsifier having a HLB generally above 7 is added after distillation is complete. Suitable emulsifiers of this type are well known to those skilled in the art. Ethoxylated alcohols are preferred.

The level of neutralization is adusted by maintaining the monomer phase temperature below 30°C and adding a metal alkali, including but not limited to sodium hydroxide, lithium hydroxide, potassium hydroxide, or nitrogen containing bases, including and not limited to ammonium hydroxide or simple amines, to the monomer phase in order to neutralize the required molar ratio of carboxylic sites measured by conventional acid-base titration or by known pH measurement systems.

It is a further feature of the invention that a suitable polymeric stabiliser surfactant is employed as a processing aid to maintain emulsion integrity through the distillation process and to provide for the final liquid polymer dispersion to be a free flowing liquid, even when it contains high levels of microparticles of the water soluble or swellable dispersed polymer.

Advantageously 0.5 to 10.0 parts, preferably 1.0 to 6.0 parts, of this polymeric surfactant is employed per part by weight (dry weight) of the ethylenically unsaturated monomer.

A preferred polymeric surfactant is a copolymer of an alkyl (math)acrylate monomer and an amino functional monomer, which may be prepared as follows:

Alkyl (meth)acrylate, amino functional monomer and a suitable oil soluble thermal initiator, for example 2,2'-azobis(2-methylbutyronitrile), are dissolved in an inert solvent, for example an aliphatic or aromatic hydrocarbon solvent such as Isopar G. This mixture is fed into a vessel containing further solvent and thermal initiator over a period of 2 to 6 hours at reaction temperatures of 80 to 90°C. The reaction is maintained at this temperature for a further two hours before being cooled and run off.

The alkyl group of the alkyl (moth)acrylate may be any suitable alkyl group, however $C_8$ to $C_{22}$ groups are preferred.

The amino functional monomer is of the general formula (1):

$$(1) \qquad CH_2=CRC(=O)-X-B-NR^1R^2,$$

wherein

R       is hydrogen or $C_1$-$C_4$alkyl,
X       is -O- or -NH-,
B       is $C_1$-$C_4$alkylene,
$R^1$     is hydrogen or $C_1$-$C_{10}$alkyl, and
$R^2$     is $C_1$-$C_{10}$alkyl.

The alkyl (meth)acrylate : amino functional monomer ratio may be between 0.5 to 8.0 : 1 on a molar basis. Preferably between 0.75 to 6.0 : 1, and most preferably between 1.0 to 4.0 : 1 on a molar basis.

The molecular weight may be determined by conventional chromatography techniques well known to those skilled in the art. Typical molecular weights may be in the range of 10,000 to 60,000, most typically in the range of 15,000 to 40,000.

Upon stirring the liquid dispersion into an aqueous system, the non-ionic surfactant converts the hydrophobic carrier into an oil-in-water emulsion. At the same time the hydrophilic polymer expands on exposure to water resulting in a smooth and rapid viscosity increase. Typically the polymer particles swell to give a microparticulate thickening system comprising polymer particles having a typical particle size in the range of 2.5 to 5.0 microns.

The inventive liquid dispersion compositions provide microparticulate thickening systems which give effective thickening to aqueous or aqueous/organic formulations at concentrations of 0.1 to 8.0 %. Preferably 1 % to 6% by weight. In addition however they combine the thickening effect of the liquid dispersion polymer with emolliency and sensorial effects delivered by the skilled selection of a suitable non-aqueous carrier fluid.

Decreasing the level of neutralization of an acrylic acid polymer of this type has been demonstrated to increase the thickening efficiency of the microparticulae thickening system in aqueous solutions. The increase in apparent viscosity is roughly linear as the level of neutralization is reduced from 100% to 30% neutralization. Below around 25% neutralization as determined by conventional acid-base titration, the solubility of the polyacrylic acid significantly reduces the effectiveness as a thickener and rheology modifier. Addition of ionic salts such as sodium chloride is known to reduce the swelling capacity of the cross-linked microparticles due to osmotic effects, but it has been demonstrated that the effect of ionic species increases at levels of neutralization below 30%, as measured by conventional acid-base titration. Therfore there is an advantage in selecting a neutralization level between 30% and 40% to provide more effective thickening in aqueous systems, oil/water emulsion and in systems containing low levels of ionic species inclding and not limited to inorganic salts, botanical extracts, proteins and similar species.

The liquid dispersion polymer compositions are compatible with a wide variety of personal care active ingredients and auxiliaries. Typical formulation examples where the polymers may be used include:

Skin Care formulations including all kind of face and body emulsions like creams, lotions, milks and sprays for caring, cleansing, deodorisation and depilation, colour cosmetics such as; liquid foundations, liquid eyeshadows, liquid blushers, lipsticks and aqueous mascaras; facial masks, lip balms, skin care formulations like body washes, all kind of shaving products; hand soaps, soap bars and soap liquids.

Hair Care formulations which include; hair conditioners, hair colourations (permanent, semi-permanent and temporary), styling gels, lotions and creams, shampoos, hair relaxers, hair perms and hair masks.

Sun Tan formulations such as: sun tan creams, lotions and sprays, sun blocks, tan accelerators, after sun creams, lotions and sprays and sunless tanning lotions or creams.

The formulation examples below merely illustrate a few representative aspects of the formulating possibilities and are not intended to be limiting in any way. All percents are percent by weight of the formulation. Viscosities are determined with a Brookfield RVT viscometer.

**Example 1:** SYNTHESIS EXAMPLE

An "aqueous" phase of water soluble components is prepared by admixing together the following components:

　　33.65 parts of acrylic acid monomer (100% concentration)
　　0.15 parts of 40% solution of pentasodiumdiethylene triaminepentaacetic acid
　　49.79 parts water
　　1.50 parts of methylene bis-acrylamide (0.5% in water)
　　14.91 parts of sodium hydroxide (47% concentration)

(During addition of sodium hydroxide maintain temperature at less than 30°C)

An "oil phase" is prepared by mixing together the following components:

　　3,99 parts of sorbitan trioleate
　　4.66 parts polymeric stabilizer (100% concentration)
　　31.16 parts of mineral white oil
　　80.19 parts of high purity dearomatised hydrocarbon solvent (such as Isopar G)

The two phases are mixed together in a ratio of 0.751 parts oil phase to 1.0 parts aqueous phase under high shear to form a water-in-oil emulsion

The resulting water-in-oil emulsion is transferred to a reactor equipped with nitrogen sparge tube, stirrer and thermometer. The emulsion is purged with nitrogen to remove oxygen Polymerisation is effected by addition of a redox couple of sodium metabisulphite and tertiary butyl Hydroperoxide.

After exotherm is complete and free monomer has been reduced by the use of thermal initiator, vacuum distillation is carried out to remove water and volatile solvent to give final polymer solids around 53.5%.

To this addition is made of 0.125 parts of a fatty alcohol ethoxylate.

The level of neutralization is adjusted to a value between 30 and 40%.

**Example 2:** Comparison of thickening efficiency at different levels of neutralization

In the table below, the thickening efficiency of polymers of the present invention prepared at a range of levels of neutralization has been compared in aqueous solutions and in a dilute ionic solution.

| % Neutralisation | Viscosity 1 % active polymer in deionised water | Viscosity 1 % active polymer in 0.1% sodium chloride solution |
| --- | --- | --- |
| 30 | 190000 | 66400 |
| 40 | 184000 | 69600 |
| 50 | 172000 | 63600 |
| 60 | 157000 | 48400 |
| 70 | 126000 | 33000 |

(continued)

| % Neutralisation | Viscosity 1 % active polymer in deionised water | Viscosity 1 % active polymer in 0.1% sodium chloride solution |
|---|---|---|
| 80 | 94900 | 11400 |
| (Measured by Brookfield RVT viscometer, Spindle 6) | | |

**Example 3:** Depilatory Cream

This formulation allows the formulation of depilatory creams based on sodium thioglycollate. Adjusting the amount of thickener used can change the product from a roll-on lotion to a viscous cream.

| | | Ingredients | Ingredients (Tradenames & Suppliers) | Amount [wt-%] |
|---|---|---|---|---|
| | 1 | Polymer of Example 1 | | 6.0 - 8.0 |
| | 2 | Paraffinum Liquidum | Kristo®l M14 (Carless) | 5.0 |
| | 3 | Glycerin | Glycerol | 2 |
| | 4 | Phenoxyethanol (and) Methylparaben (and) Ethylparaben (and) Isobutylparaben (and) Propylparaben | Phenonip (Clariant) | 0.20 |
| | 5 | Parfum | Fragrance | 0.7 |
| | 6 | Petrolatum | Vaseline (Richardson-Vicks) | 2.0 |
| | 7 | Thioglycollic Acid | | 3.0 |
| | 8 | Water | | to 100 |

Additionally, sodium hydroxide is added to adjust the pH-value in the range of 12 -13.

Method:

1    Weigh (6) into a beaker and warm to 40°C
2    Weigh all ingredients except (1) into a separate beaker and mix well
3    Add (6) with stirring.
4    Adjust the pH of the mix to between 12 and 13 with sodium hydroxide, stirring continuously
5    Add the required amount of (1) with gentle stirring, continuing to stir until smooth
6    Adjust pH to between 12 and 13 with dropwise addition of sodium hydroxide if required.

Typical Properties:

Appearance:    smooth low to high viscosity cream
Viscosity:    9000 - 45000 cPs
pH:    12-13

**Example 4:** Facial Moisturiser

This formulation contains a combination of light emollients, squalane and silicones to provide good rub-in characteristics and excellent after feel. The presence of the sunscreen provides a low level of sun protection for daily use.
The polymer is included as a thickener and emulsifier for the oil phase and allows cold-process emulsification.

In addition the mineral oil carrier phase will provide additional moisturisation properties.

| | Ingredients | Ingredients (Tradenames & Suppliers) | Amount [wt-%] |
|---|---|---|---|
| 1 | Water | | to 100 |
| 2 | Polymer of Example 1 | | 2.0 |
| 3 | Coco-caprylate/Caprate | Cetiol LC® (Cognis) | 2.5 |
| 4 | Squalane | Cetiol SQ® (Cognis) | 2.0 |
| 5 | Hexyl Laurate | Cetiol A® (Cognis) | 2.0 |
| 6 | Ethylhexyl Palmitate | Estol 1543® (Uniqema) | 2.0 |
| 7 | Dimethicone | Silicone Fluid 200 (Dow Coming) | 2.5 |
| 8 | Ethylhexyl Methoxycinnamate | Tinosorb OMC® (Ciba Specialty Chemicals) | 5.0 |
| 9 | Phenoxyethanol (and) Methylparaben (and) Ethylparaben (and) Isobutylparaben (and) Propylparaben | Phenonip® (Clariant) | 0.20 |
| 10 | Parfum | Fragrance | 0.20 |

Method:

Weigh all ingredients except (2) into a clean, dry beaker
Initiate stirring and add (2)
Continue to stir until viscous and homogeneous

Typical Properties:

Appearance:   smooth viscous cream
Viscosity:    25000 - 35000 cPs
pH:           5-6

**Example 5:** Body Moisturiser (Cream)

This formulation is based on a traditional cream containing fatty alcohols and esters. The polymer provides emulsification at low levels of incorporation and improves the stability of the external phase.

| | Ingredients | Ingredients (Tradenames & Suppliers) | Amount [wt-%] |
|---|---|---|---|
| 1 | Water | | to 100 |
| 2 | Polymer or Example 1 | | 1.0 |
| 3 | Stearyl Alcohol | Crodacol S95EP® (Croda Chemicals) | 5.0 |
| 4 | Cetyl Alcohol | Crodacol C90EP® (Croda Chemicals) | 5.0 |
| 5 | Dimethicone | Silicone Fluid 200 (Dow Coming) | 5.0 |
| 6 | Cotearyl Stearate | Estol 3709CSS® (Uniqema) | 2.0 |
| 7 | Glycerin | Glycerol | 2.0 |
| 8 | Propylene Glycol | Propylene Glycol | 2.0 |

(continued)

| | | Ingredients | Ingredients (Tradenames & Suppliers) | Amount [wt-%] |
|---|---|---|---|---|
| 9 | | Phenoxyethanol (and) Methylparaben (and) Ethylparaben (and) Isobutylparaben (and) Propylparaben | Phenonip® (Clariant) | 0.20 |
| 10 | | Parfum | Fragrance | 0.20 |

Weigh ingredients (1), (7) and (8) into a clean dry beaker and heat to 80°C
Weigh ingredients (3) - (6) into a separate clean dry beaker and heat to 80°C
Add oil phase to water phase with good stirring and initiate cooling
Add ingredient (2) at < 50°C, ingredients (9) and (10) at < 30°C
Continue to stir until viscous and homogeneous

Typical Properties:

| Appearance: | smooth viscous cream |
|---|---|
| Viscosity: | 35000 - 45000 cPs |
| pH: | 5-6 |

**Example 6:** After Sun Lotion/Cream

| | Ingredients | Ingredients (Tradenames & Suppliers) | Amount [wt-%] |
|---|---|---|---|
| 1 | Water | | to 100 |
| 2 | Prunus Dulcis | Sweet Almond Oil (AE Connock) | 4.50 |
| 3 | Kola Nut Extract | Actiphyte Kola Nut (Active Organics) | 5.00 |
| 4 | Aloe Barbadenis | Aloe Vera (Active Organics) | 2.00 |
| 5 | Polymer of Example 1 | | 3.25 |
| 6 | Aqua & Panthenol & Caprylic/Capric Triglyceride & polysorbate 80 & Lecithin | Tinoderm P® (Ciba Specialty Chemicals) | 1.25 |
| 7 | Phenoxethanol & Methyl & Propylparaben & 2-Bromo-2-Nitropropane -1.3-Diol | Nipaguard BPX® (Nipa Laboratories) | 0.20 |

Method:

Add 1 to a beaker, initiate agitation then add 2, 3 , 4, 6 and 7 mixing well for approximately 5 to 10 minutes.
After increasing stirrer speed add 5 slowly, continuing to stir slowly for about 5 to 10 minutes until viscous and homogeneous.

Typical Properties

| Appearance: | flowable lotion |
|---|---|
| Viscosity: | 5,000 - 8,000 cPs |
| pH: | 5.0 - 6.5. |

**Example 7:** Silicone Spray Conditioner

This is a light conditioner suitable for everyday use and contains light silicones and humectants. The polymer assists the incorporation of the fragrance into a largely aqueous formulation as well as providing viscosity and good application characteristics.

|  | | Ingredients | Ingredients (Tradenames & Suppliers) | Amount [wt-%] |
|---|---|---|---|---|
| **1** | | Water | | to 100 |
| **2** | | Polymer of Example 1 | | 1.0 |
| **3** | | Amodimethicone (and) Cetrimonium Chloride (and) Trideceth-12 | Silicone Fluid 949 Cationic (Dow Coming) | 5.0 |
| **4** | | Cyclomethicone | Silicone Fluid 235 (Dow Coming) | 1.0 |
| **5** | | Oleth-20 | Volpo N20® (Croda Chemicals) | 1.0 |
| **6** | | Imidazolidinyl Urea | Germall 115® (ISP) | 0.2 |
| **7** | | Parfum | Fragrance | 0.20 |

Method:

Weigh all ingredients except (2) into a clean, dry beaker
Initiate stirring and add (2)
Continue to stir until homogeneous

Typical Properties

Appearance:   sprayable lotion
Viscosity    2500 - 6000 cPs
pH:        5.0-6.0.

**Claims**

1. A process for the preparation of an aqueous dispersion polymer composition comprising a hydrophilic, water soluble or swellable polymer with a neutralization level of from 55 to 100%, a non-aqueous carrier phase and an oil-in-water surfactant, ***characterized in that*** the hydrophilic, water soluble or swellable polymer is prepared by reverse phase emulsion polymerization of hydrophilic monomers in a hydrophobic liquid phase and converting the hydrophobic carrier into an oil-in-water emulsion.

2. A process according to claim 1, ***characterized in that*** the polymer is prepared by reverse phase emulsion polymerization by adding 1 part by weight (dry weight) of at least one aqueous ethylenically unsaturated monomer, optionally including a sequesterant and a crosslinking ethylenically unsaturated monomer, into 1 to 3 parts by weight of a hydrophobic liquid comprising at least in part a non-aqueous carrier fluid and containing 0.1 to 0.2 parts of at least one conventional water-in-oil emulsifier having a HLB-value below 9.0 and optionally 0.5 to 10.0 parts of a polymeric stabilizer surfactant.

**Patentansprüche**

1. Verfahren zur Herstellung einer wässerigen Dispersionspolymerzusammensetzung umfassend ein hydrophiles, wasserlösliches oder -quellbares Polymer mit einem Neutralisationsgrad von 55 bis 100 %, eine nicht-wässrige Trägerphase und ein Öl-in-Wasser-Tensid, **dadurch gekennzeichnet, dass** das hydrophile, wasserlösliche oder -quellbare Polymer durch Umkehrphasen-Emulsionspolymerisation von hydrophilen Monomeren in einer hydrophoben wässrigen Phase hergestellt wird und der hydrophobe Träger in eine Öl-in-Wasser Emulsion umgewandelt wird.

2. Verfahren nach Anspruch 1, **dadurch gekennzeichnet, dass** das Polymer durch Umkehrphasen-Emulsionspolymerisation hergestellt wird durch Zusetzen von 1 Gewichtsteil (Trockengewicht) von mindestens einem wässrigen ethylenisch ungesättigten Monomer, wahlweise umfassend einen Komplexbildner und ein vernetzendes ethylenisch ungesättigtes Monomer, in 1 bis 3 Gewichtsteile einer hydrophoben Flüssigkeit, umfassend mindestens teilweise ein nichtwässriges Trägerfluid und 0,1 bis 0,2 Teile von mindestens einem herkömmlichen Wasser-in-Öl Emulgator mit einem HLB-Wert unter 9,0 und wahlweise 0,5 bis 10,0 Teile eines polymeren Stabilisationstensids.

**Revendications**

1. Un procédé de préparation d'une composition polymère en dispersion aqueuse comprenant un polymère hydrophile, gonflable ou hydrosoluble, présentant un taux de neutralisation de 55 à 100%, une phase de support non-aqueuse et un agent tensioactif huile-dans-l'eau, *caractérisé en ce que* le polymère hydrophile, gonflable ou hydrosoluble, est préparé par polymérisation en émulsion en phase inverse de monoméres hydrophiles dans une phase liquide hydrophobe et conversion du support hydrophobe dans une émulsion huile-dans-l'eau.

2. Un procédé selon la revendication 1, *caractérisé en ce que* le polymère est préparé par polymérisation en émulsion en phase inverse par addition de 1 partie en poids (poids à sec) d'au moins un monomère à insaturation éthylénique en phase aqueuse, incluant éventuellement un agent séquestrant et un monomère à insaturation éthylénique de réticulation, dans 1 à 3 parties en poids d'un liquide hydrophobe comprenant au moins en partie un fluide de support non aqueux et contenant 0,1 à 0,2 parties d'au moins un émulsifiant eau-dans-l'huile conventionnel présentant une valeur HLB inférieure à 9.0 et éventuellement 0,5 à 10,0 parties d'un agent tensioactif stabilisant polymère.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4677152 A

- US 4628078 A